# EUROPEAN PATENT APPLICATION

(11) **EP 2 932 986 A1**
(43) Date of publication of application: **21.10.2015**
(21) Application number: 15163586.9
(22) Date of filing: 14.04.2015
(51) Int. Cl.: A61L 2/22, A61L 9/14, B05B 1/14

(54) **MACHINE AND METHOD FOR ENVIRONMENTAL SANITISATION USING A NEBULIZED SANITISING SOLUTION CONTAINING HYDROGEN PEROXIDE AND SILVER NITRATE**

(30) Priority: 16.04.2014 IT MI20140713
(71) Applicant: Medical Device Factory S.R.L., 70032 Bitonto (BA) (IT)
(72) Inventor: Rucci, Giuseppe, 70032 BITONTO (BA) (IT)
(74) Representative: Bianchetti Bracco Minoja S.r.l.

(57) **Abstract**

A machine (10) for environmental sanitisation comprising a system of dispensing and nebulisation (12) of a sanitising solution (SS) in an environment (AM) to be sanitised, wherein the sanitising solution, used by the sanitisation machine, is constituted specifically by an aqueous solution of hydrogen peroxide (H₂O₂) and silver salts or nitrate (AgNO₃), and wherein the system of dispensing and nebulisation (12), included in the sanitisation machine, is configured in such a way as to dispense and nebulise the sanitising solution (SS) with a certain concentration, in the environment to be sanitised (AM), comprised between 3 ml/m³ and 6 ml/ m³. Advantageously the system of dispensing and nebulisation (12) of the sanitisation machine (10) comprises a nebuliser assembly (13), of the multi-nozzle type and in particular constituted by four nebulisation nozzles (13'), apt to nebulise uniformly, at 360°, the sanitising solution (SS) in the environment (AM) to be sanitised. The sanitisation machine can operate in a totally automatic manner to perform a cycle of complete sanitisation or a cycle of preventive sanitisation, and allows all the various phases of the process of sanitisation of an environment to be programmed and kept under control, thanks to a dedicated program and a practical user interface.

## Description

### Field of the invention

The present invention relates in general to the field of environmental sanitisation in order to prevent and impede the onset and the development of infections caused by the presence of bacteria or similar pathogens, and in particular its object is a machine or equipment for sanitisation suitable for diffusing and nebulising an appropriate sanitising solution or composition in an environment to be sanitised.

Moreover the object of the present invention is a corresponding method for environmental sanitisation and a corresponding sanitising solution.

It is emphasised that the invention was developed through research that involved a major part of testing and which had, as the reference framework, healthcare, both public and private, an area in which, as is known, the infections that may relate to the respective hospital structures constitute a problem of fundamental importance, in various respects - ethical, safety and economic.

In order to have an idea of the importance of this problem it is sufficient to consider that the infections which occur inside hospital structures, of public and private healthcare, are the cause of a number of deaths, every year, which is higher than that caused by road accidents.

In particular these dangerous infections are caused by the presence on inert surfaces and on the walls of the environments and the rooms of these hospital structures of micro-organisms, agents of infections, such as bacteria of the Enterococcus, Escherichia, Klebsiella, Pseudomonas, Acinetobacter and Staphylococcus type, which precisely can survive on these surfaces and walls, thus feeding the environment-operator-patient chain of contagion.

Therefore it has always been essential, in order to combat, prevent and impede the development of such infections, often favoured by inadequate hygienic conditions, to resort to methods, systems, equipment and machines, such as that which is the object of the present invention, aimed at abating the bacterial, viral and fungal load in the environments and in the rooms of interest, and in particular hospital ones, with the aid of specific products and sanitising and disinfectant compositions.

### Background of the invention and state of the art

As illustrated above, various types of machinery, equipment, installations and systems in general are already present in the art and in use for some time for environment or environmental sanitisation by the use of specific disinfectant and/or sanitising compositions and products, which, in order to increase the effects and the effectiveness, are appropriately nebulised and diffused in the environment to be sanitised.

Therefore these known machines for environmental sanitisation are usually provided with their own devices for the dispensing of the disinfectant substance and its nebulisation and diffusion in the environment to be sanitised.

It has been recorded however how both the traditional sanitisation and disinfecting systems and methods, taken as a whole, and the respective systems of nebulisation of the sanitising substance and also the specific sanitising compositions and substances that are used in these known systems of sanitisation, have, despite their consolidated application, many limits and disadvantages.

In particular, from the point of view of effectiveness and efficiency, these known systems of sanitisation do not always allow all the surfaces to be treated to be reached easily and effectively, especially the hidden ones, which therefore may become a reservoir of bacteria and micro-organisms, and therefore potential cause of infections.

Moreover the nebulisation systems currently used in known sanitisation systems are usually based on the wet nebulisation of sanitising and disinfectant products, with the drawback that in this way there is a certain environmental impact and the treated surfaces are left wet.

Again these known nebulisation systems generally require that the rooms and environments subjected to cleaning and sanitisation are of the watertight closure type, which entails long times, after having performed the cleaning and disinfection, before the rooms can be usable again.

Moreover, from the point of view of safety, the systems and the processes of sanitisation and disinfection currently known and in use have the relevant limitation of being operator-dependent, i.e. requiring the use of staff, exposing them in this way to multiple risks, such as that of inhaling of toxic products and of skin irritation.

In brief these known systems of sanitisation and the relative systems of nebulisation have numerous critical features in some fundamental points connected to inadequate safety for humans, to long times of nebulisation, to insufficient uniformity of distribution and diffusion of the sanitising product in the environment to be sanitised and a certain invasiveness of the same sanitising product when used in environments in which electromedical appliances are present.

### Summary of the invention

Therefore the present invention, as part of the current context as previously outlined and having therefore, as reference, the needs and the problems, posed and noted in this context, of preventing and impeding in the most effective way possible the onset of bacterial infections, has as primary object that of proposing and making an appliance or machine for environmental sanitisation which constitutes a substantial improvement with respect to the sanitisation systems and machines currently known and in use, and in particular offers improved performances and results in terms of efficacy for sanitising and disinfecting environments, and is moreover characterised by a fully automatic working, so as to avoid the use of qualified personnel and in any case exonerate the operator from any manual operation.

A second object of the present invention, in any case connected to the previous one, is to define a new sanitising substance or composition or solution for environmental sanitisation which is characterised by a greater efficacy with respect to the currently known and used compositions and solutions, and to associate and combine therefore this new and more effective sanitising composition or solution with an improved sanitisation machine, using specifically this new composition or solution, so as to go beyond and obtain decidedly superior results with respect to those which can be obtained with the current sanitisation machines and systems.

Finally a further object of the present invention, this also connected to the previous ones, is to develop both a new machine for environmental sanitisation and a new sanitising solution or composition, usable in combination, so as to achieve a combined system, for environmental sanitisation, which offers significantly improved and more effective performances with respect to traditional systems, in which these improved performances and this increased efficacy are verified and demonstrated in an incontrovertible and objective way by appropriate experimental tests.

The above objects can be considered fully achieved by the environmental sanitisation machine, by the corresponding method of sanitisation and by the sanitising composition having the features defined respectively by the independent claims 1, 7 and 10.

Particular embodiments of the present invention are moreover defined by the dependent claims.

As anticipated and illustrated in greater detail here below in the description the guiding idea at the basis of the present invention was to provide a system or machine or device for environmental sanitisation, which has been given the trade name of SANISIM, which was more automated, more effective, safer, more practical to use and also involved shorter sanitisation times with respect to those which can be obtained with the already known sanitisation systems on the market, in particular through the use of a new sanitising solution, which has been given the trade name of SANISIM SOLUTION, which in turn shows greater efficiency and improved performances with respect to the sanitising compositions and solutions currently known.

As mentioned previously, both the SANISIM sanitising machine or device and the SANISIM SOLUTION used in the SANISIM machine represent the result of advanced research and have been the subject of an extensive and thorough experimentation aimed at ascertaining their innovative features and performances.

In particular the SANISIM machine was designed by carefully selecting every single component used, so that it was of superior quality and involved easy maintenance.

In this way a sanitisation machine or system was developed and perfected, precisely the SANISIM, the object of the present invention, which allows the environment to be sanitised materially in a completely automatic way, thus without the need to use qualified personnel.

At the same time, the SANISIM machine allows the changing and programming, in this case by qualified personnel, of the various parameters of the sanitisation process, so as to adapt the system to any working situation and need.

Furthermore, the SANISIM system allows its functions to be remote-piloted at a controlled distance.

The SANISIM system is also provided with a system of self-calibration for the management of the quantity of the sanitising substance or liquid to be dispensed and nebulised in the environment or room to be sanitised.

Finally, in SANISIM, the nebulisation and the diffusion, in the environment to be sanitised, of the sanitising substance or product, is performed in a uniform manner, at 360°.

### Advantages of the invention

There are numerous advantages, in part already mentioned, of the sanitisation machine which is the object of the present invention i.e. of the SANISIM, such as those listed here below purely by way of a non-limiting example:
- easy installability in the environment to be sanitised;
- extremely short times of sanitisation;
- easy programmability;
- maintenance and running costs;
- total lack of risks for the operator;
- extremely effective environmental sanitisation, with disinfection in the sanitised environment of 99.99% of viruses, bacteria, fungi and spores, as shown by extensive experimental tests.

### Brief description of the drawings

These and other objects, features and advantages of the present invention will be made clearer and evident by the following description of one of its preferred embodiments, given by way of a non-limiting example, with reference to the accompanying drawings, in which:
Fig. 1 is a general block diagram of a machine or equipment or device, according to the present invention, for environmental sanitisation by nebulisation of a sanitising solution in an environment to be sanitised;
Figs. 2A- 2C are drawings and photographic images of a nebuliser of the sanitising solution which is used by the sanitisation machine of Fig. 1 for sanitising the environment;
Fig. 3 is a diagram of a pneumatic system included in the sanitisation machine of Fig. 1 and associated with the nebuliser of Figs. 2A-2C;
Fig. 4 is a flow diagram that illustrates a method, according to the present invention, for environmental sanitisation, in particular which can be performed with the sanitisation machine of Fig. 1; and
Figs. 5A and 5B are photographic images of a SANISIM machine, in accordance with the present invention and actually realised, for environmental sanitisation.

### Description of a preferred embodiment of the invention

Referring to the drawings a machine or equipment or device for environmental sanitisation, in accordance with the present invention, is denoted overall by 10.

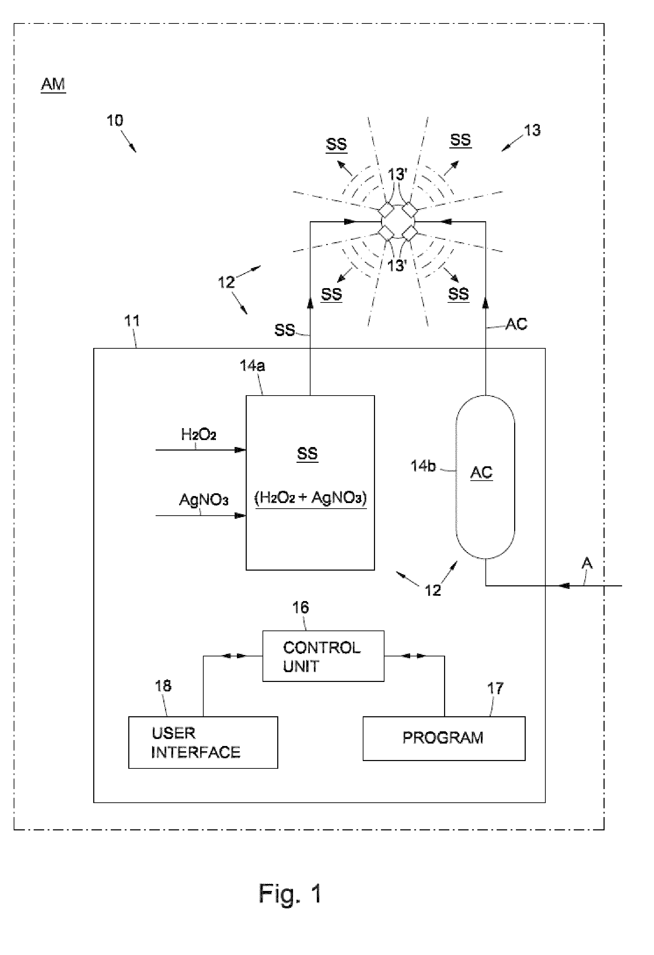

As shown schematically in Fig. 1, the sanitisation machine or device 10 is provided to be placed in the interior of an environment to be sanitised, denoted by AM, in order to diffuse there a sanitising composition or solution, in turn denoted by SS, and thus preventing the onset and the development of infections caused by the presence in the environment AM, in particular on the respective walls, of bacteria and/or similar pathogens.

The environment AM, to be sanitised by means of the machine 10, can be of various kinds, and for example be made up of a hospital room, such as an operating theatre, a consulting room, a room for the hospitalisation of patients or another kind, in which, as stated previously, it is necessary to create hygienic conditions such as to prevent and inhibit the onset of infections caused by the presence in the environment AM of bacteria and/or similar pathogens.

In detail, the sanitisation machine or device 10, also called by the commercial name SANISIM, comprises:
- a fixed support structure, denoted by 11 and schematised with a rectangular block in Fig. 1, to support the various parts of the sanitisation machine 10; and
- a system of dispensing and nebulisation, associated with the fixed structure 11 and denoted as a whole by 12, to dispense and nebulise, in the environment AM to be sanitised, the sanitising solution or composition SS, also known under the trade name SANISIM SOLUTION.

According to a first feature which qualifies the present invention and distinguishes it from known sanitisation systems and methods, the sanitising solution SS or SANISIM SOLUTION, which is used by the sanitisation machine 10 or SANISIM for sanitising the environment AM, is made up of an aqueous solution containing ≤ 6% by weight of hydrogen peroxide H₂O₂ and a quantity comprised between 40 and 60 mg/l, preferably 47 mg/ml, of silver nitrate AgNO₃ or silver salts.

Therefore the sanitising solution SS exploits the oxidising action of the hydrogen peroxide which, as is known, is an oxidising agent capable of eliminating the pathogens with which it comes into contact through the hydrolysis of the free radicals, and is active in respect of a wide range of micro-organisms, such as bacteria, fungi, viruses and spores, so that it is considered an effective and safe disinfectant.

In particular, the hydrogen peroxide exerts its sanitising and disinfectant action by generating an oxidation of the lipid structures of the membranes, and an alteration of the ribosomes and of the nucleic acids.

Moreover the sanitising solution SS exploits the cationic action of the silver salts, which in turn assist and complement the bactericidal action exerted by the hydrogen peroxide, deactivating the pathogens through the inhibition of the protein synthesis and ensuring at the same time the extension in time of the biocidal action, preventing the proliferation thereof.

In particular, as is known, the silver salts generate an inversion with consequent alteration of the polarity of the membrane and an inhibition of the protein synthesis.

Again, according to a further feature apt to qualify and distinguish the present invention from the prior art, the system of dispensing and nebulisation 12, included in the sanitisation machine 10, is configured so as to dispense and nebulise the sanitising solution SS with a concentration, in the environment AM to be sanitised, comprised between 3 ml/m³ and 6 ml/m³.

For this purpose the dispensing and nebuliser system 12 comprises a nebuliser or nebuliser assembly, denoted overall by 13, which in turn is apt to nebulise the sanitising solution SS, in the environment AM to be sanitised, in the form of micro-drops or dry mist, which has the feature and the advantage of not wetting, in which the dimensions of the micro-drops have a typical Gaussian distribution with an average size comprised between 6 µm to 9 µm.

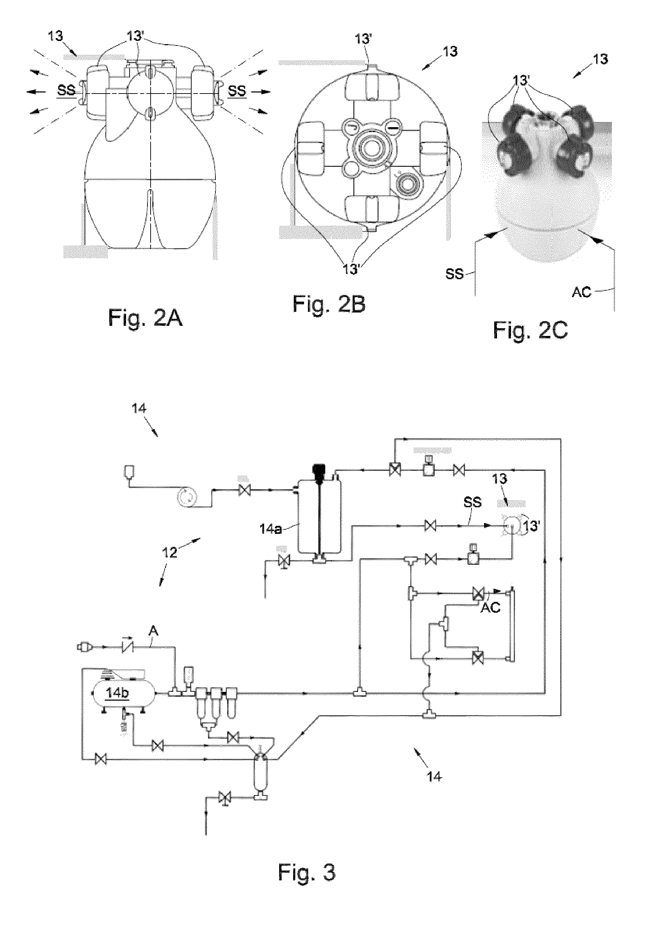

The nebuliser assembly 13 is provided to receive both the sanitising solution SS, composed of H₂O₂ and AgNO₃, and compressed air, denoted by AC, aimed to nebulise the sanitising solution SS, and, advantageously, as shown in Figs. 2A-2C, 13 is of the multi-nozzle type, and in particular comprises four nebulisation nozzles, denoted by 13', which are arranged uniformly around a central axis so as to be apt to dispense and nebulise the sanitising solution SS, evenly over 360°, in the environment AM to be sanitised.

The sanitisation machine 10 also comprises a pneumatic circuit, denoted overall by 14 and shown in detail in Fig. 3, associated with the dispensing and nebulisation system 12 and the respective nebuliser assembly 13.

More particularly the pneumatic circuit 14 comprises a tank 14a for the preparation of the sanitising solution SS, in the specific percentage required, as defined previously, of hydrogen peroxide H₂O₂ and silver salts or silver nitrate AgNO₃, and a compressor 14b apt to receive air A from the outside environment and compress it, to then feed the nebuliser assembly 13 with the compressed air AC.

Finally the sanitisation machine 10 comprises an electronic control unit 16, for example made up of a PLC, which has the function of governing and controlling the general working of the machine 10 and which, for this purpose, is associated with a specific dedicated program 17, containing the instructions which define this working, and an interface 18, by means of which a user can command, control and interact with the sanitisation machine 10.

For example, advantageously, this interface 18 can be of a graphic type and include a contact screen, i.e. of the touch screen type, apt to allow an immediate and easy dialogue between the sanitisation machine 10 and the respective operator.

### Working of the machine for environmental sanitisation

The working of the sanitisation machine 10, or SANISIM, for performing the process of sanitisation of an environment AM, comprises various phases in sequence, defined by the program 17 associated with the control unit 16, and in particular those listed here below:
a) starting of the sanitisation machine and entering of the parameters and of the input data by the operator;
b) checking of the parameters and of the data entered;
c) launch and automatic execution of the cycle or process of sanitisation, with dispensing and nebulisation of the nebulising substance in the environment to be sanitised according to the times and the methods established by the respective program;
d) end of the sanitisation process or cycle and printing of a report with the results of this sanitisation process.

In particular, in phase (a), the control unit 16 of the SANISIM calculates automatically the quantity, expressed in ml, of the sanitising solution SS to be nebulised, and the respective nebulisation time, expressed in minutes, on the basis of the data supplied by the operator, such as in particular the volumetry, i.e. the width, length and height, of the room to be sanitised.

Advantageously, during the working, the sanitisation machine 10 is programmed, in the case of obstruction of one of the nebulisation nozzles 13' of the nebuliser assembly 13, to intervene automatically so as to increment automatically the time of dispensing and nebulisation of the sanitising solution SS dispensed and nebulised by the other nebulisation nozzles 13' of the nebulisation assembly 13, so as to compensate the reduction, due to this obstruction, of the quantity of the sanitising solution SS in the environment AM to be sanitised and therefore always observe the correct concentration of the sanitising solution SS nebulised in the environment to be sanitised.

As a general rule the SANISIM is programmed to perform two different processes or cycles of sanitisation, obtaining the same end results, that is to say complete sanitisation, defined by a complete cycle of sanitisation of the environment to be sanitised, or a preventive sanitisation, defined by a maintenance cycle, as illustrated here below.

### Complete sanitisation

Complete sanitisation is performed by nebulising in the environment to be treated and sanitised a quantity of sanitising solution or liquid in a concentration equal to 6 ml/m³ in the same environment to be sanitised.

Usually this complete cycle of sanitisation is launched in those environments which have never been subjected previously to sanitising treatment and also whenever it is necessary to perform complete sanitisation in relation to the environmental conditions of the site, to the quality and quantity features of the bacterial load present, to the frequency of the work programmed and to the agreements made with the user.

### Preventive sanitisation

Preventive sanitisation is instead performed by nebulising into the environment to be sanitised a quantity of sanitising liquid or solution, according to a concentration, in the environment to be sanitised, equal to 3 ml/m³.

In this case the maintenance cycle, defining this preventive sanitisation, is usually performed in those environments that have already been previously subjected to a complete cycle of sanitisation and in any event within a predefined sanitisation plan with regular interventions in time in relation to the environmental conditions of the site, the quality and quantity features of the bacteria load present and commercial agreements made with the user.

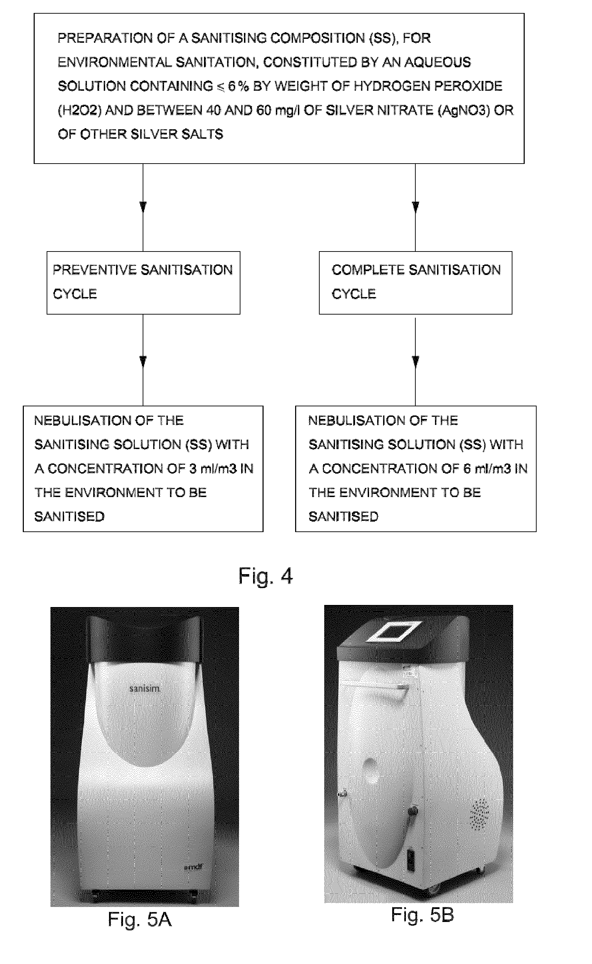

For clarity the block flow diagram of Fig. 4 schematises a method for environmental sanitisation, as described previously, in accordance with the present invention and which can be realised in particular with the sanitisation machine 10, wherein this method comprises an initial phase of preparation of a sanitising composition SS, constituted by an aqueous solution containing ≤ 6% of hydrogen peroxide (H₂O₂) and a quantity comprised between 40 and 60, preferably 47, of mg/l of silver nitrate (AgNO₃) or of another silver salt, and a phase of nebulisation of this solution, in an environment AM to be sanitised, in the form of micro-drops or dry mist with the micro-drops having an average size, as part of a Gaussian distribution, comprised between 6 µm and 9 µm, and wherein this phase of nebulisation is performed by dispensing and nebulising the sanitising solution SS according to a concentration of 3 ml/m³ in the environment AM to be sanitised, as part of a preventive sanitisation cycle, or by dispensing and nebulising the sanitising solution SS according to a concentration of 6 ml/m³, as part of a cycle of complete sanitisation of the environment AM to be sanitised.

It is therefore clear, from what is described, that the present invention reaches in full the objects that had been set and offers a new machine for environmental sanitisation, based on the use of a new sanitising solution or substance, which goes well beyond and overcomes the limits, the disadvantages and the performances of the systems, of the machines and of the methods of sanitisation currently known and in use, and in particular is suitable for sanitising in reduced time and in an extremely practical, simple and safe manner a wide variety of critical rooms and environments, such as for example those which are part of a hospital structure and which therefore, due to the bacterial load present in them, could determine, if not appropriately sanitised, the onset of dangerous infections in patients.

Without prejudice to the basic concepts of the present invention it is clear that changes and further improvements may be made to the sanitisation machine described here without thereby departing from the scope of the same invention.

### EXPERIMENTAL TESTS

As already underlined several times, the sanitisation machine which is the object of the present invention was developed and perfected through a series of in-depth experimental tests and checks which confirmed its innovative features and performances and the important advantages compared with the environmental sanitisation systems and machines currently known and in use.

Therefore, in order to give more complete information and as confirmation of the innovative features and advantages of the present invention, the results of some of the experimental tests and trials are to be given here below which were performed on a prototype of the SANISIM sanitisation machine which uses the sanitising solution SANISIM SOLUTION, in order to ascertain effectively the bacterial presence, expressed in cfu (colony-forming units) following a process or cycle of environmental sanitisation performed with this combined SANISIM + SANISIM SOLUTION system.

### TEST 1

Type of test: preventive sanitisation with a concentration equal to 3 ml/m³ of the sanitising solution in the environment to be sanitised.
Nebulisation time: 576 seconds (9.6')
Time of exposure to the nebulised sanitising solution: 1' - 15' - 30' - 60'

| BACTERIAL STRAIN | 10⁵ | 10⁴ | 10³ | 10² | 10 | 1 |
|---|---|---|---|---|---|---|
| Pseudomonas aeruginosa ATCC 27853 | | | | | | |
| Control | 700,000 cfu/ml | 70,000 cfu/ml | 7,000 cfu/ml | 700 cfu/ml | 69 cfu/ml | 7 cfu/ml |
| 1' | 35 cfu/ml | 17 cfu/ml | 0 cfu/ml | 0 cfu/ml | 0 cfu/ml | 0 cfu/ml |
| 15' | 1 cfu/ml | 1 cfu/ml | 0 cfu/ml | 0 cfu/ml | 0 cfu/ml | 0 cfu/ml |
| 30' | 0 cfu/ml | 0 cfu/ml | 0 cfu/ml | 0 cfu/ml | 0 cfu/ml | 0 cfu/ml |
| 60' | 0 cfu/ml | 0 cfu/ml | 0 cfu/ml | 0 cfu/ml | 0 cfu/ml | 0 cfu/ml |

| Escherichia coli ATCC 25922 | | | | | | |
|---|---|---|---|---|---|---|
| Control | 350,000 cfu/ml | 34,000 cfu/ml | 3,400 cfu/ml | 300 cfu/ml | 29 cfu/ml | 3 cfu/ml |
| 1' | 42 cfu/ml | 10 cfu/ml | 0 cfu/ml | 0 cfu/ml | 0 cfu/ml | 0 cfu/ml |
| 15' | 0 cfu/ml | 0 cfu/ml | 0 cfu/ml | 0 cfu/ml | 0 cfu/ml | 0 cfu/ml |
| 30' | 0 cfu/ml | 0 cfu/ml | 0 cfu/ml | 0 cfu/ml | 0 cfu/ml | 0 cfu/ml |
| 60' | 0 cfu/ml | 0 cfu/ml | 0 cfu/ml | 0 cfu/ml | 0 cfu/ml | 0 cfu/ml |
| | | | | | | |

| BACTERIAL STRAIN | 10⁵ | 10⁴ | 10³ | 10² | 10 | 1 |
|---|---|---|---|---|---|---|
| Staphylococcus aureus ATCC 25923 | | | | | | |
| Control | 130,000 cfu/ml | 13,000 cfu/ml | 1,300 cfu/ml | 126 cfu/ml | 19 cfu/ml | 4 cfu/ml |
| 1' | 36 cfu/ml | 4 cfu/ml | 1 cfu/ml | 0 cfu/ml | 0 cfu/ml | 0 cfu/ml |
| 15' | 0 cfu/ml | 0 cfu/ml | 0 cfu/ml | 0 cfu/ml | 0 cfu/ml | 0 cfu/ml |
| 30' | 0 cfu/ml | 0 cfu/ml | 0 cfu/ml | 0 cfu/ml | 0 cfu/ml | 0 cfu/ml |
| 60' | 0 cfu/ml | 0 cfu/ml | 0 cfu/ml | 0 cfu/ml | 0 cfu/ml | 0 cfu/ml |

### TEST 2

Type of test: complete sanitisation with a concentration, in the environment to be sanitised, equal to 6 ml/m³ of the sanitising solution.
Nebulisation time: 1170 seconds (19,5')
Time of exposure to the nebulised sanitising solution: 1' - 15' - 30' - 60'

| BACTERIAL STRAIN | 10⁵ | 10⁴ | 10³ | 10² | 10 | 1 |
|---|---|---|---|---|---|---|
| Pseudomonas aeruginosa ATCC 27853 | | | | | | |
| Control | 700,000 cfu/ml | 70,000 cfu/ml | 7,000 cfu/ml | 700 cfu/ml | 69 cfu/ml | 7 cfu/ml |
| 1' | 9 cfu/ml | 0 cfu/ml | 0 cfu/ml | 0 cfu/ml | 0 cfu/ml | 0 cfu/ml |
| 15' | 0 cfu/ml | 0 cfu/ml | 0 cfu/ml | 0 cfu/ml | 0 cfu/ml | 0 cfu/ml |
| 30' | 0 cfu/ml | 0 cfu/ml | 0 cfu/ml | 0 cfu/ml | 0 cfu/ml | 0 cfu/ml |
| 60' | 0 cfu/ml | 0 cfu/ml | 0 cfu/ml | 0 cfu/ml | 0 cfu/ml | 0 cfu/ml |
| | | | | | | |

| BACTERIAL STRAIN | 10⁵ | 10⁴ | 10³ | 10² | 10 | 1 |
|---|---|---|---|---|---|---|
| Escherichia coli ATCC 25922 | | | | | | |
| Control | 350,000 cfu/ml | 34,000 cfu/ml | 3,400 cfu/ml | 300 cfu/ml | 29 cfu/ml | 3 cfu/ml |
| 1' | 2 cfu/ml | 10 cfu/ml | 0 cfu/ml | 0 cfu/ml | 0 cfu/ml | 0 cfu/ml |
| 15' | 0 cfu/ml | 0 cfu/ml | 0 cfu/ml | 0 cfu/ml | 0 cfu/ml | 0 cfu/ml |
| 30' | 0 cfu/ml | 0 cfu/ml | 0 cfu/ml | 0 cfu/ml | 0 cfu/ml | 0 cfu/ml |
| 60' | 0 cfu/ml | 0 cfu/ml | 0 cfu/ml | 0 cfu/ml | 0 cfu/ml | 0 cfu/ml |

| Staphylococcus aureus ATCC 25923 | | | | | | |
|---|---|---|---|---|---|---|
| Control | 130,000 cfu/ml | 13,000 cfu/ml | 1,300 cfu/ml | 126 cfu/ml | 19 cfu/ml | 4 cfu/ml |
| 1' | 3 cfu/ml | 0 cfu/ml | 0 cfu/ml | 0 cfu/ml | 0 cfu/ml | 0 cfu/ml |
| 15' | 0 cfu/ml | 0 cfu/ml | 0 cfu/ml | 0 cfu/ml | 0 cfu/ml | 0 cfu/ml |
| 30' | 0 cfu/ml | 0 cfu/ml | 0 cfu/ml | 0 cfu/ml | 0 cfu/ml | 0 cfu/ml |
| 60' | 0 cfu/ml | 0 cfu/ml | 0 cfu/ml | 0 cfu/ml | 0 cfu/ml | 0 cfu/ml |

As can be observed from the data given above, in both tests, concerning respectively a cycle of preventive sanitisation and a cycle of complete sanitisation, the combined system SANISIM + SANISIM SOLUTION has given excellent results and has shown that it is very effective in eradicating all the bacterial strains tested.

Moreover the various experimental tests which were performed have allowed it to be ascertained that the sanitising solution SANISIM SOLUTION, used in the SANISIM machine according to the present invention and constituted specifically by an aqueous solution containing ≤ 6% by weight of hydrogen peroxide H₂O₂ and around 47 mg/l of silver salts or nitrate AgNO₃, fully complies with the regulations in force and therefore apt to sanitise the environments and rooms as provided by these regulations.

### FURTHER CERTIFICATION TESTS

An illustration will also be given here below, again for the purpose of supplying information which is as complete as possible, of the tests which were performed in the two phases provided by current regulations, at accredited laboratories and centres, and the relative results obtained, to obtain certification of the machine for environmental sanitisation in accordance with the present invention and the corresponding method of sanitisation.

In particular these tests, carried out as mentioned at accredited centres and laboratories, were aimed at assessing the disinfectant activity of the SANISIM SOLUTION product, based on ≤6% hydrogen peroxide and silver ions obtained by diluting hydrogen peroxide stabilized to 48% with bi-distilled water.

Efficacy tests were also carried out as provided in the two phases of the approval process for class IIa medical devices disinfectants.

### PHASE 1 AND RELATIVE DATA

The tests of this phase 1 were performed in the laboratory on bacterial strains at a concentration equal for all of 5Log₁₀ prepared in physiological solution and subjected to treatment with 1 ml of solution of SANISIM SOLUTION diluted with water at different concentrations: with hydrogen peroxide ≤ 6% and ≤ 3% with silver ions.

The results obtained in this phase 1 are summarised in the following table.

| Table 1: efficacy tests phase 1 | | | | |
|---|---|---|---|---|
| Activity | Strain | Concentration of micro-organisms | Concentration of product used | Abatement noted |
| Bactericidal for pathogenic, coagulase-positive, gram-positive bacteria | Staphylococcus aureus ATCC 25923 | 10⁵ cfu/ml | <6% Hydrogen peroxide+silver ions | 5Log₁₀ |
| Bactericidal for pathogenic, coagulase-positive, gram-positive bacteria | Staphylococcus aureus ATCC 25923 | 10⁵ cfu/ml | <3% Hydrogen peroxide+silver ions | 2,5Log₁₀ |
| Bactericidal for bacteria of faecal origin, glucuronidase-positive, gram-negative | Escherichia coli ATCC 25922 | 10⁵ cfu/ml | <6% Hydrogen peroxide+silver ions | 5Log₁₀ |
| Bactericidal for bacteria of faecal origin, glucuronidase-positive, gram-negative | Escherichia coli ATCC 25922 | 10⁵ cfu/ml | <3% Hydrogen peroxide+silver ions | 2,5Log₁₀ |
| Bactericidal, gram-negative, oxidase-positive, mainly pulmonary pathogen | Pseudomonas aeruginosae ATCC 27853 | 10⁵ cfu/ml | <6% Hydrogen peroxide+silver ions | 5Log₁₀ |
| Bactericidal, gram-negative, oxidase-positive, mainly pulmonary pathogen | Pseudomonas aeruginosae ATCC 27853 | 10⁵ cfu/ml | <3% Hydrogen peroxide+silver ions | 2,5Log₁₀ |
| Bactericidal for pathogenic, coagulase-positive, gram-positive, antibiotic resistant bacteria | Staphylococcus aureus MRSA ATCC 43300 | 10⁵ cfu/ml | <6% Hydrogen peroxide+silver ions | 5Log₁₀ |
| Bactericidal, faecal origin, gram-positive, resistant to extreme environmental conditions, antibiotic resistant | Enterococcus faecalis VRE ATCC 51299 | 10⁵ cfu/ml | <6% Hydrogen peroxide+silver ions | 5Log₁₀ |
| Bactericidal, nosocomial pathogen, resistant in the environment, antibiotic resistant | Acinetobacter baumannii ATCC 19606 | 10⁵ cfu/ml | <6% Hydrogen peroxide+silver ions | 5Log₁₀ |
| Bactericidal, gram-negative, pathogen for the immunocompromised, growing antibiotic resistance | Klebsiella pneumoniae ATCC 700603 | 105 cfu/ml | <6% Hydrogen peroxide+silver ions | 5Log₁₀ |

### PHASE 2: FIRST TEST PART AND RELATIVE DATA

This first part of tests performed in the laboratory during phase 2 was aimed at evaluating the bactericidal activity of SANISIM SOLUTION.

In particular in these tests each bacterial strain was tested with the product, diluted with water to three different concentrations and in dirty conditions simulated in the laboratory, for a contact time of 5 minutes and at a constant temperature of 20°C.

The results obtained in this first test part, in phase 2, are summarised in Table 2 below.

| Table 2: phase 2 efficacy tests (first part of the tests) | | | | | | |
|---|---|---|---|---|---|---|
| Activity | Method/protoco 1 to be tested | Species | Concentratio n of micro-organisms | Abatement found as a function of the concentration of the SANISIM solution and in dirty conditions | | |
| | | | | 6% with silver ions | 3% with silver ions | 1% with silver ions |
| Bactericidal for pathogenic, coagulase-positive, gram-positive bacteria | UNI EN 13727:2014 | Staphylococcu s aureus ATCC 25923 | 10⁵ cfu/ml | 5Logl 0 | 3 Log10 | 1,5 Log1 0 |
| Bactericidal for bacteria of faecal origin, glucuronidase -positive, gram-negative | UNI EN 13727:2014 | Escherichia coli ATCC 25922 | 10⁵ cfu/ml | 5Log1 0 | 3Log1 0 | 1,5 Log1 0 |
| Bactericidal, gram-negative, oxidase-positive, mainly pulmonary pathogen | UNI EN 13727:2014 | Pseudomonas aeruginosae ATCC 27853 | 10⁵ cfu/ml | 5Log1 0 | 3 Log10 | 1,5 Log1 0 |
| Bactericidal for pathogenic, coagulase-positive, gram-positive, antibiotic resistant bacteria | UNI EN 13727:2014 | Staphylococcus aureus MRSA ATCC 43300 | 10⁵ cfu/ml | 5Log10 | 2,5 Log₁₀ | 0,5 Log₁₀ |
| Bactericidal, faecal origin, gram-positive, resistant to extreme environmental conditions, antibiotic resistant | UNI EN 13727:2014 | Enterococcus faecalis VRE ATCC 51299 | 10⁵ cfu/ml | 5Log10 | 2,5 Log10 | 0,5 Log10 |
| Bactericidal, nosocomial pathogen, resistant in the environment, antibiotic resistant | UNI EN 13727:2014 | Acinetobacter baumannii ATCC 19606 | 10⁵ cfu/ml | 5Log10 | 3 Log₁₀ | 1.5 Log₁₀ |
| Bactericidal, gram-negative, pathogen for the immuno compromised, growing antibiotic resistance | UNI EN 13727:2014 | Klebsiella pneumoniae ATCC 700603 | 10⁵ cfu/ml | 5Log10 | 3 Log₁₀ | 1.5 Log₁₀ |

### PHASE 2: SECOND TEST PART AND RELATIVE DATA

This second part of tests, during phase 2, was also carried out in the laboratory.

In particular a test suspension of each bacterium, mixed with a solution of interfering substances, was inoculated on two surfaces of stainless steel and dried. Then on one of the two surfaces a prepared sample of the product SANISIM SOLUTION diluted in water was applied, so as to cover the dried film. On the other surface only water was instead deposited. The contact on both surfaces took place for 5 minutes at 20°C. Subsequently both surfaces were immersed in a neutralisation solution so that the action of the disinfectant was immediately neutralised. Then from each surface the bacterial suspension was recovered and its viability evaluated quantitatively.

The results obtained in this second test part, during phase 2, are summarised in table 3 below.

| Table 3: phase 2 efficacy tests (second part of the tests) | | | | | |
|---|---|---|---|---|---|
| Activity | Method/protocol to be tested | Species | Concentration of micro-organisms | Abatement of bacteria on the surface after 5 minutes of contact at 20°C in dirty conditions | |
| | | | | Water | 6% with silver ions |
| Bactericidal for pathogenic, coagulase-positive, gram-positive bacteria | UNI EN 13697:2001 | Staphylococcus aureus ATCC 25923 | 10⁵ cfu/ml | 1Log₁₀ | 5Log₁₀ |
| Bactericidal for bacteria of faecal origin, glucuronidase-positive, gram-negative | UNI EN13697:2001 | Escherichia coli ATCC 25922 | 10⁵ cfu/ml | 1Log₁₀ | 5Log₁₀ |
| Bactericidal, gram-negative, oxidase-positive, mainly pulmonary pathogen | UNI EN 13697:2001 | Pseudomonas aeruginosae ATCC 27853 | 10⁵ cfu/m | 1Log₁₀ | 5Log₁₀ |
| Bactericidal for pathogenic, coagulase-positive, gram-positive, antibiotic resistant bacteria | UNI EN13697:2001 | Staphylococcus aureus MRSA ATCC 43300 | 10⁵ cfu/ml | 1Log10 | 5Log₁₀ |
| Bactericidal, faecal origin, gram-positive, resistant to extreme environmental conditions, antibiotic resistant | UNI EN 13697:2001 | Enterococcus faecalis VRE ATCC 51299 | 10⁵ cfu/ml | 1Log10 | 5Log₁₀ |
| Bactericidal, nosocomial pathogen, resistant in the environment, antibiotic resistant | UNI EN13697:2001 | Acinetobacter baumannii ATCC 19606 | 10⁵ cfu/ml | 1Log₁₀ | 5Log₁₀ |
| Bactericidal, gram-negative, pathogen for the immunocompromised, growing antibiotic resistance | UNI EN 13697:2001 | Klebsiella pneumoniae ATCC 700603 | 10⁵ cfu/ml | 1Log₁₀ | 5Log₁₀ |

Tests were also carried out in which account was taken of the contact time of the bacterial suspension with the SANISIM SOLUTION.

These further tests, of which some data are given here below, have also demonstrated and confirmed the remarkable effectiveness of the combined system SANISIM + SANISIM SOLUTION in breaking down the bacterial flora in all the strains tested.

| Activity | Method/ protocol to be tested | Species | Time of contact Sanisim system | Abatement noted |
|---|---|---|---|---|
| Bactericidal for pathogenic, coagulase-positive, gram-positive bacteria | NF T 72-281 UNI EN 1040:2006 | Staphylococcus aureus ATCC 25923 | 1 minute | 5Log₁₀ |
| Bactericidal for bacteria of faecal origin, glucuronidase-positive, gram-negative | NF T 72-281 UNI EN 1040:2006 | Escherichia coli ATCC 25922 | 1 minute | 5Log₁₀ |
| Bactericidal, gram-negative, oxidase-positive, mainly pulmonary pathogen | NF T 72-281 UNI EN 1040:2006 | Pseudomonas aeruginosae ATCC 27853 | 1 minute | 5Log₁₀ |
| Bactericidal for pathogenic, coagulase-positive, gram-positive, antibiotic resistant bacteria | NF T 72-281 UNI EN 1040:2006 | Staphylococcus aureus MRSA ATCC 43300 | 15 minutes | 5Log₁₀ |
| Bactericidal, faecal origin, gram-positive, resistant to extreme environmental conditions, antibiotic resistant | NF T 72-281 UNI EN 1040:2006 | Enterococcus faecalis VRE ATCC 51299 | 15 minutes | 5Log₁₀ |
| Bactericidal, nosocomial pathogen, resistant in the environment, antibiotic resistant | NF T 72-281 UNI EN 1040:2006 | Acinetobacter baumannii ATCC 19606 | 1 minute | 5Log₁₀ |
| Bactericidal, gram-negative, pathogen for the immunocompromised, growing antibiotic resistance | NF T 72-281 UNI EN 1040:2006 | Klebsiella pneumoniae ATCC 700603 | 1 minute | 5Log₁₀ |
| Sporicidal, gram-positive, thermophilic bacteria | NF T 72-281 UNI EN 1040:2006 | Geobacillus stearothermophilus ATCC 7953 | - | 5Log₁₀ |

In conclusion, the numerous tests carried out, such as described previously, including those for obtaining certification, and the related experimental data, clearly demonstrate that the sanitisation performed with the SANISIM system or machine or device and the corresponding sanitising composition SANISIM SOLUTION, the object of the present invention, applied observing the times and the methods of dispensing of the sanitising composition SANISIM SOLUTION indicated previously, are very effective and such as to have positive effects for abating all the bacterial strains tested.

### STABILITY OF THE SANISIM SOLUTION PRODUCT

Moreover it is stressed that the sanitising solution SANISIM SOLUTION, in addition to the significant bactericidal effects illustrated previously, is characterised by and has the advantage of a high stability both in time and under varying operating conditions in which the solution is used to sanitise an environment.

In particular, as it was possible to ascertain through specific tests, the product SANISIM SOLUTION maintains intact the title of hydrogen peroxide and of silver at least in the timespan of one year from production.

Again the SANISIM SOLUTION, with a percentage of hydrogen peroxide of 6% by weight, or in any case close to this value, has passed positively the tests and the specific stability tests, to which it was subjected, at the variation of the electrical conductivity or the salt content of the water used to perform the dilution of the hydrogen peroxide and of the silver salts.

In particular, as shown by the following table, in these stability tests the conductivity value was varied from 5 µS/cm up to a value of 1000 µS/cm, with times of permanence of the SANISIM SOLUTION in these conductivity conditions from 0 up to 24 hours, always obtaining a positive outcome.

| Test | Conductivity (uS/cm) | Time of permanence (hours) | Hydrogen peroxide title (%) | Outcome of test |
|---|---|---|---|---|
| 1 | 5 | 0 | 6.1 | Positive |
| 2 | 5 | 1 | 6.1 | Positive |
| 3 | 5 | 12 | 6.0 | Positive |
| 4 | 5 | 24 | 6.0 | Positive |
| 5 | 100 | 0 | 6.1 | Positive |
| 6 | 100 | 1 | 6.0 | Positive |
| 7 | 100 | 12 | 6.0 | Positive |
| 8 | 100 | 24 | 5.6 | Positive |
| 9 | 1000 | 0 | 6.1 | Positive |
| 10 | 1000 | 1 | 6.0 | Positive |
| 11 | 1000 | 12 | 6.0 | Positive |
| 12 | 1000 | 24 | 6.0 | Positive |

The stability tests performed on the SANISIM SOLUTION at the variation of the temperature, pH and time of exposure to the light also gave a positive outcome.

Finally it is pointed out that the nebulised product SANISIM SOLUTION, used in the environmental sanitisation, continues its action in time and is effective even after the contact time necessary for eradicating the pathogens, so as to significantly delay the onset of a new bacterial load.

## Claims

1. Machine (10) for environmental sanitisation comprising:
- a sanitising composition (SS), for environmental sanitisation, constituted by an aqueous sanitising solution containing ≤ 6% by weight of hydrogen peroxide (H₂O₂) and a quantity comprised between 40 and 60 mg/l, preferably 47 mg/l, of silver nitrate (AgNO₃) or of other silver salts, and
- a system of dispensing and nebulisation (12) of said sanitising solution (SS) in an environment (AM) to be sanitised,
wherein said machine for the sanitisation (10) is configured to dispense and nebulise said sanitising solution (SS) with a concentration, in the environment to be sanitised (AM), comprised between 3 ml/m³ and 6 ml/m³, and
wherein said dispensing and nebulisation system (12), included in the sanitisation machine (10), comprises a nebuliser assembly (13), of the multi-nozzle type (13'), which is configured in such a way as to dispense and nebulise uniformly, in the environment (AM) to be sanitised, said sanitising solution (SS), in the form of micro-drops or dry mist with the micro-drops which have an average size, as part of a Gaussian distribution, comprised between 6 µm and 9 µm.

2. Machine (10) for environmental sanitisation according to claim 1, wherein said nebuliser assembly (13) comprises at least four nebulisation nozzles (13') distributed evenly around a central axis so as to dispense and nebulise the sanitising solution (SS) uniformly over 360° and wherein said nebuliser assembly (13) is apt to receive both the sanitising solution (SS, (H₂O₂ + AgNO₃)) and compressed air (AC) aimed at nebulising the sanitising solution (SS).

3. Machine (10) for environmental sanitisation according to claim 1 or 2, further comprising a system of preparation (14) of the sanitising solution (SS), a storage tank (14a) of the same sanitising solution (SS), once prepared, and a compressor (14b) apt to receive air (A) from the outside environment in order to compress it, which are associated with said dispensing and nebulisation system (12) of the sanitising solution (SS) in the environment (AM) to be sanitised.

4. Machine (10) for environmental sanitisation according to any one of the preceding claims, further comprising a user interface (18), of the graphic type, to allow the user to program and control the working of the sanitisation machine (10).

5. Machine (10) for environmental sanitisation according to any one of the preceding claims, wherein said machine, in the case of obstruction of one of the nebulisation nozzles (13') of said nebuliser assembly (13), of the multi-nozzle type, is configured in such a way as to compensate for the decrease, due to such an obstruction, of the quantity of sanitising solution (SS) in the environment to be sanitised and to increase correspondingly the time of dispensing and nebulisation of the sanitising solution dispensed and nebulised by the other nebulisation nozzles (13') of the nebulisation assembly (13) so as to always comply with the correct concentration of the sanitising solution (SS) nebulised in the environment to be sanitised.

6. Machine (10) for environmental sanitisation according to any one of the preceding claims, wherein said machine is configured in such a way as to perform a complete sanitisation cycle, dispensing and nebulising said sanitising solution (SS) with a concentration of 6 ml/m³ in the environment to be sanitised, or, alternatively, a cycle of preventive sanitisation, dispensing and nebulising said sanitising solution (SS) with a concentration of 3 ml/m³ in the environment (AM) to be sanitised.

7. Method for environmental sanitisation comprising a phase of nebulisation of a sanitising composition (SS) according to a certain concentration in an environment (AM) to be sanitised, wherein the sanitising composition is made up of an aqueous sanitising solution containing ≤ 6% hydrogen peroxide (H₂O₂) and a quantity comprised between 40 and 60, preferably 47, of mg/l of silver nitrate (AgNO₃) or of another silver salt, and wherein the sanitising solution (SS) is nebulised by a nebuliser assembly (13), of the multi-nozzle type (13'), which is configured in such a way as to dispense and nebulise uniformly, in the environment (AM) to be sanitised, the sanitising solution (SS) in the form of micro-drops or dry mist with the micro-drops having an average size, as part of a Gaussian distribution, comprised between 6 µm and 9 µm.

8. Method for environmental sanitisation according to claim 7, wherein said nebulisation phase is performed by dispensing and nebulising said sanitising solution (SS) with a concentration of 3 ml/m³ in the environment (AM) to be sanitised, as part of a preventive sanitisation cycle, or by dispensing and nebulising said sanitising solution (SS) with a concentration of 6 ml/m³, as part of a complete cycle of sanitisation of the environment (AM) to be sanitised.

9. Method for environmental sanitisation according to claim 7 or 8, wherein the sanitising solution (SS) is dispensed uniformly over 360°, by means of an appropriate nebuliser (13, 13'), in the environment (AM) to be sanitised.

10. Sanitising solution, (SS) apt to be nebulised in an environment (AM) by means of a sanitisation machine according to claims 1-6, said solution consisting of an aqueous solution containing ≤ 6% by weight of hydrogen peroxide (H₂O₂) and a quantity comprised between 40 and 60 mg/l, preferably 47 mg/l, of silver nitrate (AgNO₃) or of a different silver salt.
